# FASCICULE DE BREVET EUROPEEN

(11) **EP 0 904 353 B1**
(45) Date de publication et mention de la délivrance du brevet: **02.07.2003**
(21) Numéro de dépôt: 97918225.0
(22) Date de dépôt: 15.04.1997
(51) Int. Cl.: C12N 5/02, C12N 5/08, C12N 5/06, C12P 21/00, C07K 14/54

(54) **PROCEDE ET MILIEU DE CULTURE DE CELLULES, COMPOSITION CELLULAIRE OBTENUE ET SES APPLICATIONS EN TANT QUE SYSTEME DE PRODUCTION ET DE MODELE D'ETUDE**
VERFAHREN UND MEDIUM ZUR ZELLZÜCHTUNG, SO ERHALTENE ZELLZUSAMMENSETZUNG UND IHRE ANWENDUNGEN ALS PRODUKTIONSSYSTEM UND ALS FORSCHUNGSMODELL
CELL CULTURE PROCESS AND MEDIUM, CELLULAR COMPOSITION OBTAINED AND ITS APPLICATION AS PRODUCTION SYSTEM AND STUDY MODEL

(30) Priorité: 16.04.1996 FR 9604709; 24.12.1996 FR 9615947
(43) Date de publication de la demande: 31.03.1999
(73) Titulaire: UNIVERSITE DE NANTES, F-45035 Nantes Cédex 01 (FR)
(72) Inventeur: LABOISSE, Christian, F-44000 Nantes (FR)
(74) Mandataire: Laget, Jean-Loup
(86) Numéro de dépôt international: FR9700668
(87) Numéro de publication internationale: WO97039109

(56) Documents cités:
- EP-A- 0 403 139
- FR-A- 2 634 784
- US-A- 4 743 552

## Description

La présente invention concerne un procédé et un milieu de culture in vitro de cellules, telles que des cellules primaires normales ou tumorales ou des lignées cellulaires, d'origine humaine ou animale, une composition cellulaire à base de cellules épithéliales gastro-intestinales et un système de production de molécules bioactives et/ou d'ARNm incluant une telle composition ainsi qu'un modèle d'étude constitué de ladite composition.

La culture de cellules quelconques d'origine humaine ou animale comprend toujours une étape d'adhérence de ces cellules à un support également quelconque. Cette étape d'adhérence est nécessaire pour obtenir un taux de survie correct des cellules tout en assurant le maintien de leurs fonctions spécialisées. A l'inverse, une absence d'adhérence des cellules à un support entraîne une mort rapide desdites cellules.

Cette étape d'adhérence pose encore un certain nombre de problèmes devant être résolus, en particulier pour certaines cellules primaires, normales ou tumorales, particulièrement fragiles. Ainsi, on ne sait toujours pas, à ce jour, maintenir en culture primaire des populations pures de cellules épithéliales gastro-intestinales alors que, parallèlement, on sait que l'épithélium intestinal constitue une extraordinaire source de peptides bioactifs qui pourraient se révéler d'un grand intérêt pour des industries telles que les industries pharmaceutiques.

Les méthodes décrites jusqu'ici pour le maintien en culture de telles populations étaient essentiellement fondées sur la reconstitution de matrices extra-cellulaires et/ou l'utilisation de milieux définis supplémentés par diverses protéines et facteurs de croissance. Certains auteurs sont même allés jusqu'à la reconstitution in vitro de la structure tridimensionnelle de la crypte intestinale sur "feeder-layer" endothélial. Cependant, toutes ces méthodes se sont révélées inopérantes en pratique du fait du très faible taux d'adhérence et, donc, de viabilité des cellules en culture, de l'absence de reproductibilité des résultats, de la trop grande complexité des méthodes mises en oeuvre et de l'absence de maintien des fonctions différenciées desdites cellules. Ces mêmes méthodes ont été utilisées pour certaines cellules normales tumorales qui posent actuellement les mêmes problèmes.

Parallèlement, la culture de cellules, en particulier de lignées cellulaires, à l'échelle industrielle, est également très dépendante de l'étape d'adhérence pour des raisons différentes de celles évoquées ci-dessus. Ainsi, par exemple, l'optimisation des conditions de culture par accélération de l'adhérence et par adhérence en masse des cellules constitue un facteur déterminant pour l'amélioration du rendement de cultures en masse réalisées sur microbilles en fermenteur par exemple. En conséquence, l'adhérence doit être réalisée dans des conditions telles qu'il soit obtenu un très fort taux d'adhérence des cellules à un support quelconque et ceci dans un temps très court. Or, aujourd'hui, les milieux de culture utilisés nécessitent plusieurs heures pour provoquer l'adhérence des cellules à un support et contiennent en outre des protéines animales qui risquent à terme d'être interdites dans ce type de milieu du fait des risques de contamination éventuelle par un virus et/ou par un agent pathogène de type prion des cellules. L'étape d'adhérence est donc une étape longue, fastidieuse et à risques, en particulier pour la production de vaccins.

Un premier but de l'invention est donc de proposer un procédé de culture in vitro de cellules quelconques qui permet de les faire adhérer rapidement et en masse à un support quelconque pour assurer leur survie.

Un autre but de l'invention est de proposer un milieu d'incubation desdites cellules simple à fabriquer et apte à induire une adhérence rapide et en masse des cellules à un support quelconque, ce milieu permettant en outre d'obtenir des populations pures de cellules n'ayant jamais pu être obtenues à ce jour.

Un autre but de la présente invention est de proposer un milieu d'incubation desdites cellules chimiquement défini exempt d'éléments, en particulier de protéines, risquant de contaminer des cellules en culture par des agents infectieux de nature virale ou autres qui pourraient être véhiculés par lesdits éléments.

Un autre but de l'invention est de proposer une composition cellulaire d'un nouveau type dont les cellules, du fait de leur adhérence en masse à un support, peuvent être maintenues en culture dans de bonnes conditions pendant une durée allant jusqu'à plusieurs jours.

Un autre but de l'invention est de fournir un système de production par l'épithélium de molécules biologiquement actives et/ou d'ARNm codant pour ces molécules, ces molécules pouvant constituer des molécules nouvelles et inventives.

Un autre but de la présente invention est encore de proposer un modèle d'étude in vitro de cellules épithéliales intestinales qui permet, du fait de l'incorporation dans ce dit modèle d'une population pure de cellules épithéliales intestinales normales, une étude in vitro desdites cellules, du mode de régulation de leur prolifération et de leur différenciation, de l'expression de leurs fonctions spécialisées et du métabolisme et de la toxicité de substances diverses qui peuvent être ingérées, notamment de médicaments et d'aliments, dans des conditions proches de celles qui seraient obtenues dans le cas d'études in vivo.

A cet effet, l'invention a pour objet un procédé de culture in vitro de cellules, telles que des cellules primaires normales ou tumorales et/ou des lignées cellulaires, d'origine humaine ou animale, caractérisé en ce qu'il comprend, avant l'étape de mise en culture dans un milieu de culture apte à assurer la survie des cellules et à maintenir leurs fonctions spécialisées, au moins une étape d'incubation transitoire pendant une période de temps prédéterminée comprise dans la plage [5 - 60] minutes desdites cellules, de préférence isolées, dans un milieu d'incubation se présentant sous forme d'une solution dont les constituants sont choisis de manière telle que la concentration calcique finale de la solution est comprise dans la plage [0 - 1 mM], de préférence [0 - 100 µM] pour provoquer une adhérence rapide et en masse proche d'un rendement d'adhérence de 100 % desdites cellules isolées à un support quelconque en contact avec ledit milieu d'incubation.

Selon un mode de mise en oeuvre préféré de l'invention, les cellules, une fois qu'elles ont adhéré à leur support, sont cultivées dans un milieu de culture dont la concentration calcique est progressivement augmentée jusqu'à une valeur prédéterminée apte à assurer la survie des cellules et à maintenir leurs fonctions spécialisées.

L'invention concerne également un milieu d'incubation de cellules, telles que des cellules primaires normales ou tumorales et/ou des lignées cellulaires d'origine humaine ou animale, pour la mise en oeuvre du procédé de culture du type précité, caractérisé en ce que le milieu, exempt de protéines d'origine animale, se présente sous forme d'une solution dont les constituants sont choisis de manière telle que la concentration calcique finale de la solution est comprise dans la plage [0 - 1 mM], de préférence [0-100 µM].

L'intérêt d'un tel milieu d'incubation est sa simplicité de fabrication, sa composition exempte d'éléments de contamination, tels que des protéines, en particulier des protéines d'origine animale.

L'invention a encore pour objet une composition cellulaire d'un nouveau type obtenue par la mise en oeuvre du procédé du type précité, caractérisée en ce qu'elle est constituée d'une population pure de cellules épithéliales gastro-intestinales primaires normales adhérées en masse à un support avant d'être cultivées dans un milieu de culture, l'adhérence audit support ayant été obtenue par incubation transitoire pendant une période de temps comprise dans la plage [5 - 60 min] de primoculture de cellules épithéliales gastro-intestinales normales isolées de mammifères dans un milieu d'incubation se présentant sous forme d'une solution dont les constituants sont choisis de manière telle que la concentration calcique finale de la solution du milieu d'incubation est comprise dans la plage [0 - 1 mM], de préférence [0 - 100 µM].

Lorsqu'elle est cultivée dans des milieux de culture appropriés, cette composition cellulaire peut être utilisée d'une part comme système de production in vitro par l'épithélium gastro-intestinal de molécules biologiquement actives telles que les facteurs de croissance, les facteurs de réparation et/ou les ARNm codant pour lesdites molécules, d'autre part comme modèle d'étude in vitro du comportement des cellules épithéliales intestinales notamment dans des domaines tels que la pharmacotoxicologie, l'immunologie, en particulier pour l'aide à la fabrication de vaccins, et la thérapie génique.

L'invention sera bien comprise à la lecture de la description suivante d'un exemple de réalisation, en référence aux dessins annexés dans lesquels :
la figure 1 représente, sous forme graphique, l'influence de la composition, en particulier de la concentration en cations, de milieux d'incubation sur l'adhérence de cellules ;
la figure 2 représente, sous forme graphique, un test comparatif de l'efficacité d'induction de l'adhérence des cellules à un support de trois milieux d'incubation différents et
la figure 3 est une courbe qui représente, au cours du temps, la quantité d'Interleukine-8 sécrétée par les cellules épithéliales intestinales humaines normales traitées ou non par un agent pathogène.

Le procédé de culture in vitro de cellules sera plus particulièrement décrit ci-après en application à la culture de cellules épithéliales gastro-intestinales. Toutefois, tout type de cellules primaires normales ou tumorales ou de lignées cellulaires peut être utilisé sous forme de suspension cellulaire pour permettre la mise en oeuvre du procédé.

Le procédé de culture in vitro de cellules primaires épithéliales gastro-intestinales décrit ci-après se décompose en quatre étapes dont seules les étapes 2 et 3 sont caractéristiques de la présente invention.

L'étape 1 consiste à obtenir des suspensions de cellules épithéliales gastro-intestinales isolées de mammifères, en particulier de l'homme adulte. Le choix de cellules humaines adultes est préféré en raison de la destination de ces cellules (études de pharmacotoxicologie, études immunologiques, etc.). Cette étape nécessite un prélèvement d'organe ou de fragment d'organe sur le mammifère et, dans le cas de l'homme, une pièce de résection obtenue au cours d'une intervention chirurgicale. L'isolement des cellules épithéliales gastro-intestinales est réalisé à partir dudit organe ou fragment d'organe prélevé. Cette étape 1 met en oeuvre des protocoles classiques largement décrits dans la littérature.

L'étape 2 se caractérise par l'ensemencement des suspensions cellulaires obtenues dans l'étape 1 dans un milieu d'incubation caractéristique de l'invention. Ce milieu d'incubation permet aux cellules d'adhérer ou d'attacher en masse et de manière rapide à un support. Cette étape d'adhérence est indispensable pour maintenir en vie lesdites cellules et maintenir leurs fonctions essentielles.

L'étape 3 consiste à remettre en culture lesdites cellules ainsi adhérées à leur support dans un milieu de culture dont on augmente progressivement la concentration calcique pour permettre aux cellules de survivre dans des conditions permettant le maintien de leurs fonctions spécialisées. Cette caractéristique est importante pour permettre une étude ultérieure du comportement desdites cellules. Le milieu de culture à la base de cette étape 3 peut être un milieu de culture dit standard utilisé pour la culture de cellules.

L'étape 4 consiste à modifier le milieu de culture, éventuellement par addition d'éléments, ou à remplacer ce milieu de culture par un autre milieu de culture pour permettre soit la production intra ou extra-cellulaire par les cellules de molécules biologiquement actives particulières, soit pour la réalisation de tests du comportement desdites cellules à l'égard d'éléments introduits dans lesdits milieux de culture ou directement dans la cellule. Cette étape 4 peut consister en la reprise de tests déjà réalisés par exemple sur des lignées cellulaires épithéliales gastro-intestinales transformées pour vérifier les résultats qui avaient été obtenus avec de telles lignées cellulaires.

Il va à présent être décrit en détail les étapes 1 à 4 de ce procédé. Les cellules primaires ou cellules de première explantation, utiles à la mise en oeuvre de l'invention, sont obtenues à partir d'organes ou de fragments d'organes du système gastro-intestinal, tels que l'intestin grêle, le côlon, l'estomac, prélevés sur des mammifères. Plus particulièrement, chez l'homme adulte, le prélèvement est réalisé sur pièce de résection chirurgicale en muqueuse saine à distance d'une tumeur.

Le prélèvement de muqueuse et l'isolement de cellules épithéliales gastro-intestinales à partir d'une telle pièce sont des techniques de dissociation non enzymatiques bien connues, notamment décrites par Ahnen et coll. (Am. J. Physiol. 254, G 610-G 621 - 1988). Le protocole opératoire décrit ci-dessous est donc cité uniquement à titre d'exemple.

Après ouverture et rinçage de la pièce de colectomie, des fragments de muqueuse sont prélevés et placés immédiatement dans une solution PBS (fabriquée par GIBCO-BRL) exempte de calcium et de magnésium mais contenant des antibiotiques et du citrate de sodium (27 mM). Ces fragments sont rincés deux fois dans cette solution appelée solution 1. La solution 1 supplémentée en EDTA 1 mM est injectée sous l'épithélium, puis la muqueuse est totalement immergée dans cette solution. L'incubation est faite sur plateau oscillant pendant 20 mn à température ambiante. Ceci favorise la dissociation de l'épithélium du reste de la muqueuse. La muqueuse est ensuite grattée délicatement avec le bord mousse d'une lame de scalpel. La suspension cellulaire est filtrée sur une toile à "bluter" de porosité voisine de 250 µm. Le filtrat est centrifugé à 1300 tr/mn pendant 10 mn. Le surnageant est éliminé et le culot cellulaire obtenu est débarrassé des hématies par aspiration douce avec une pipette Pasteur. Ce culot cellulaire est à nouveau lavé avec un milieu qui, de préférence, est le milieu d'incubation qui sera décrit ci-après. Ce lavage est suivi d'une nouvelle centrifugation. Le surnageant est à nouveau éliminé. Le culot de centrifugation ainsi obtenu est constitué de cellules épithéliales gastro-intestinales dites primaires. Ces cellules peuvent présenter un très faible taux de contamination par des cellules non épithéliales.

Le culot cellulaire ainsi obtenu permet la mise en oeuvre de l'étape 2, appelée étape d'adhérence des cellules à un support, qui est l'étape caractéristique de la présente invention. Le culot cellulaire obtenu est remis en suspension dans un milieu d'incubation, lui-même placé en contact avec un support quelconque. Par support quelconque, il faut entendre tout support classiquement utilisé pour la culture cellulaire. Ainsi, à titre d'exemple, la suspension cellulaire peut être répartie soit dans des boîtes de Pétri, soit dans des puits de plaques de culture multipuits (plaque 96 puits fabriquée par Nunc ou Falcon), soit dans des flacons de culture en verre ou en plastique. On note ici l'un des premiers intérêts de ce procédé. En effet, il n'est pas nécessaire de faire subir au support un prétraitement par des protéines d'adhérence ou par du sérum contrairement aux procédés de culture mis en oeuvre jusqu'à maintenant. On supprime ainsi tout risque de contamination des cellules cultivées, en particulier par un agent viral ou bactérien. Il n'est pas non plus nécessaire d'adjoindre à cet ensemencement la présence d'autres cellules.

Les cellules sont laissées à incuber environ 30 mn à 37°C dans une étuve à CO₂. Ce temps est le temps moyen nécessaire pour une adhérence maximale des cellules au support. Toutefois, en fonction de la qualité des cellules du culot cellulaire utilisé, on constate que l'on peut incuber lesdites cellules isolées dans ledit milieu d'incubation pendant une période de temps prédéterminée généralement comprise dans la plage [5 - 60 minutes] pour obtenir un rendement d'adhérence des cellules au support qui peut être proche de 100 %. Dans certains cas, l'adhérence est extrêmement rapide et ne nécessite que 5 à 10 mn d'incubation dans ledit milieu. Dans d'autres cas, elle nécessite plusieurs dizaines de minutes pour permettre l'obtention d'un rendement d'adhérence permettant une mise en oeuvre optimale des étapes ultérieures du procédé. Les valeurs de rendements obtenues varient entre 60 et 100 % avec une moyenne de 90 ± 8 %. Dans tous les cas, cependant, cette étape d'incubation est une étape transitoire qui sert à la transition entre l'étape de prélèvement et d'obtention du culot de cellules et l'étape de mise en culture.

Plusieurs milieux d'incubation présentant tous les mêmes caractéristiques peuvent être utilisés dans cette étape 2. Le milieu d'incubation doit, dans tous les cas, se présenter sous forme d'une solution dont les constituants sont choisis de manière telle que la concentration calcique finale de la solution est comprise dans la plage [0 - 1 mM], de préférence [0 - 100 µM], pour provoquer une adhérence rapide et en masse desdites cellules isolées au support en contact avec ledit milieu d'incubation. Le milieu d'incubation préféré est un milieu à pH et osmolarité contrôlés qui se présente sous forme d'une solution comprenant au moins un élément nutritif et un ou plusieurs sels choisis de manière telle qu'au moins l'un des cations est présent dans la solution à une concentration au moins égale à 50 % de la concentration cationique finale de ladite solution. Les inventeurs pensent que cette caractéristique de milieu de culture permet d'induire un état de dépolarisation membranaire des cellules qui favorise l'adhérence de ces dernières au support. Ainsi, à titre d'exemple, ce milieu d'incubation comprend au moins, outre un élément nutritif tel que du glucose et/ou de la créatine et éventuellement un antioxydant tel que de la taurine, au moins un ou plusieurs sels tels que des sels à base de potassium ou de sodium, le ou lesdits sels étant choisis de la manière décrite ci-dessus.

Un exemple de composition préférée dudit milieu d'incubation peut être tel que suit :
KCl : 30-90 mM
K-glutamate : 1-10 mM
KH₂PO₄ : 10-50 mM
MgSO₄ : 1-10 mM
CaCl₂ : 0-0,1 mM
EGTA : 0-1 mM
créatine : 1-10 mM
taurine : 1-30 mM
glucose : 5-30 mM
Hepes : 10 mM
pH ajusté à 7,2 avec KOH.

Ce milieu sera appelé milieu induisant l'adhérence (M.I.A.) (KCl). Dans ce cas, le cation potassium constitue l'espèce cationique prédominante.

On peut également imaginer une composition du milieu d'incubation telle que suit :
NaCl : 30-90 mM
Na-glutamate : 1-10 mM
NaH₂PO₄ : 10-50 mM
MgSO₄ : 1-10 mM
CaCl₂ : 0-0,1 mM
EGTA : 0-1 mM
créatine : 1-10 mM
taurine : 1-30 mM
glucose : 5-30 mM
Hepes : 10 mM
pH ajusté à 7,2 avec KOH.

Ce milieu sera appelé milieu induisant l'adhérence -1 (M.I.A.-1) (NaCl). Dans ce cas, le cation sodium constitue l'espèce cationique prédominante.

On peut également utiliser un milieu d'incubation dont la composition est telle que suit :
KCl : 15 - 45 mM
NaCl : 15 - 45 mM
KH₂PO₄ : 10 - 50 mM
MgSO₄ : 1-10 mM
CaCl₂ : 0-0,1 mM
EGTA : 0-1 mM
créatine : 1-10 mM
taurine : 1-30 mM
glucose : 5-30 mM
Hepes : 10 mM
pH ajusté à 7,2 avec KOH.

Ce milieu sera appelé milieu induisant l'adhérence -2 (M.I.A.-2)(KCl/NaCl).

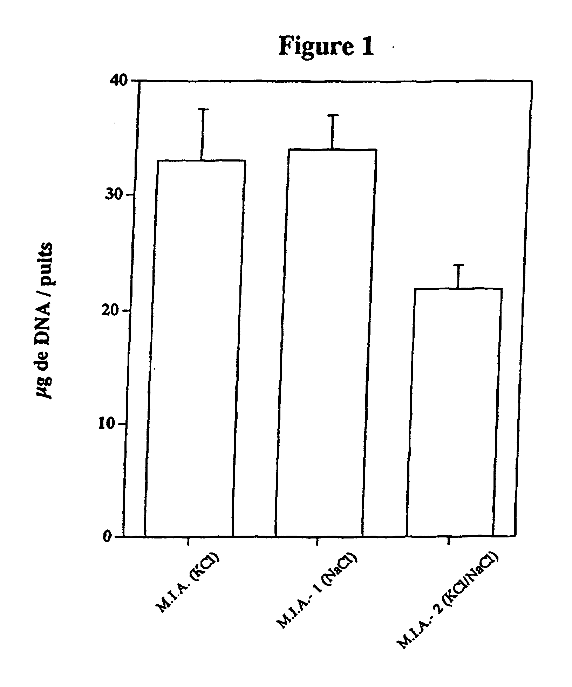

La figure 1 montre l'efficacité de ces milieux pour provoquer une adhérence des cellules à leur support. Ce graphique a été établi à partir de cellules épithéliales coliques humaines normales qui ont été ensemencées à densité égale en plaques 6 puits (Costar) dans les milieux M.I.A. (KCl), M.I.A.-1 (NaCl), M.I.A.-2 (KCl/NaCl) et laissées à incuber pendant 30 mn. Après incubation, les cellules non adhérentes ont été éliminées par lavage et les cellules adhérentes ont été quantifiées par dosage de l'ADN dans les puits par la Méthode de Burton modifiée par Taylor et Coll. (Journal of Steroid biochemistry 20 (1984) 1083-1088). On constate ainsi que la composition monocationique du milieu d'incubation induit un rendement d'adhérence plus élevé des cellules à leur support.

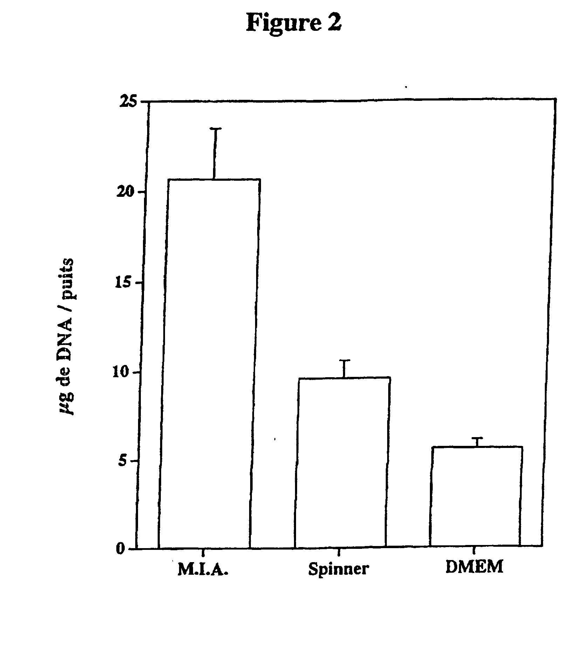

Une autre expérience résumée sous forme de graphique (figure 2) a été réalisé de manière à tester l'efficacité de différents milieux d'incubation sur l'adhérence des cellules épithéliales gastro-intestinales, y compris des milieux de culture standard. Ainsi, des cellules épithéliales coliques humaines normales ont été ensemencées à densité égale en plaques 6 puits (Costar) dans les milieux suivants : DMEM (milieu de culture standard fabriqué par Gibco-BRL), Spinner-MEM (milieu de culture standard sans calcium fabriqué par Gibco-BRL) et le milieu M.I.A. (KCl) tel que décrit ci-dessus. Après incubation de 30 mn, les cellules non adhérentes ont été éliminées par lavage et les cellules adhérentes ont été quantifiées par dosage d'ADN dans les puits de manière analogue à ce qui a été décrit ci-dessus. La figure 2 démontre clairement que le milieu M.I.A. (KCl) est particulièrement efficace en terme d'adhérence des cellules à leur support. Le milieu DMEM ne permet pas quant à lui de mettre en oeuvre les étapes ultérieures en raison de la faible concentration des cellules adhérées à leur support. En effet, cette faible adhérence induit une faible reproductibilité du système. Le milieu Spinner-MEM exempt de calcium pourrait être éventuellement utilisé comme milieu d'incubation.

Les cellules ainsi obtenues peuvent, une fois adhérées à leur support, être mises en culture dans un milieu de culture apte à assurer la survie des cellules et à maintenir leurs fonctions spécialisées. En effet, si cette incubation devait être prolongée pendant une période de temps supérieure à celle précisée ci-dessus, on constaterait rapidement un taux de mortalité élevé desdites cellules. Il est donc nécessaire, une fois l'adhérence des cellules à leur support réalisée, de remplacer le milieu d'incubation par un milieu de culture approprié. Les inventeurs ont constaté également que, si les cellules, une fois adhérées à leur support, sont cultivées dans un milieu de culture dont la concentration extra-cellulaire calcique est élevée, la mortalité des cellules est très élevée. On a constaté également que, si l'adhérence avait été réalisée dans de mauvaises conditions, par exemple avec un milieu d'incubation tel que le milieu Spinner ou le milieu DMEM, ces cellules ne retrouvent pas, par la suite, lorsqu'elles sont placées en culture dans un milieu de culture approprié, l'ensemble de leurs fonctions. De ce fait, une étude complète de ces cellules n'est pas possible. En conséquence, pour obtenir par exemple des cellules en culture dont les caractéristiques sont les plus proches de cellules normales in vivo, ces cellules, une fois adhérées à leur support, sont cultivées dans un milieu de culture dont la concentration extra-cellulaire calcique est progressivement augmentée jusqu'à une valeur prédéterminée apte à assurer la survie des cellules et à maintenir leurs fonctions spécialisées.

Ainsi, à titre d'exemple, une fois l'adhérence des cellules obtenue, le milieu M.I.A. (KCl) peut être éliminé et remplacé par un milieu de culture, appelé milieu A, dont la base est le milieu Spinner-MEM (fabriqué par GIBCO-BRL) contenant en sus 0,08 mM CaCl₂ + insuline (5.10⁻⁸M) + transferrine (10 µg/ml) + EGF (facteur de croissance épithéliale) (5ng/ml). Après incubation de 30 mn à 37°C en étuve à CO₂, un tiers du volume de ce milieu est enlevé et remplacé par le milieu DMEM (fabriqué par GIBCO-BRL), en maintenant constante la concentration en insuline, EGF et transferrine. Le but de ces changements successifs est d'augmenter progressivement la concentration calcique de la solution jusqu'à une concentration extra-cellulaire calcique finale d'environ 1 mM. Bien évidemment, cette concentration extra-cellulaire calcique finale peut être supérieure à cette valeur. La concentration calcique extra-cellulaire, dans la plupart des milieux de culture, est de l'ordre de 1,8 mM.

En général, le milieu de culture apte à la survie desdites cellules est un milieu dont la base nutritive comprend, outre des éléments nutritifs et un ou plusieurs sels, au moins de l'insuline et/ou de la transferrine et/ou de la toxine cholérique et/ou le facteur de croissance épithéliale E.G.F. et/ou des extraits hypophysaires et/ou de la dexaméthasone et/ou du sélénium et/ou des albumines sériques. La composition de ce milieu peut varier à l'infini en fonction de la destination des cellules ainsi cultivées. La composition cellulaire ainsi obtenue est constituée d'une population pure de cellules épithéliales gastro-intestinales adhérées à un support.

Ce procédé de culture présente donc à la fois l'avantage de permettre la survie dans de bonnes conditions d'un grand nombre de cellules épithéliales gastro-intestinales mais en outre d'obtenir une population pure de ces cellules. Ainsi, à titre d'exemple, on obtient des rendements d'adhérence de l'ordre de 90 % et on obtient donc des concentrations cellulaires de 10⁶ cellules, chiffre jamais atteint à ce jour. Ce qui pourra faciliter la mise en oeuvre de l'étape 4. Les cellules ainsi cultivées peuvent être maintenues dans le milieu de culture pendant plusieurs jours et elles peuvent se prêter à diverses manipulations qui font l'objet de l'étape 4.

L'étape 4 peut se présenter sous des formes diverses et variées fonction des choix de l'utilisateur. En effet, l'utilisateur peut décider d'utiliser la composition cellulaire obtenue en tant que système de production in vitro de molécules biologiquement actives et/ou d'ARNm. Ainsi, à titre d'exemple, la population pure de cellules épithéliales gastro-intestinales adhérées à un support est cultivée dans un ou plusieurs milieux de culture appropriés et est utilisée comme système de production in vitro par l'épithélium gastro-intestinal de molécules biologiquement actives et/ou d'ARNm. Par molécules biologiquement actives, on entend notamment les facteurs de croissance et les facteurs de réparation. En effet, comme cela a déjà été précisé ci-dessus, l'épithélium constitue une extraordinaire source de peptides bioactifs, tels que par exemple des peptides à activité anti-bactérienne (défensines), des peptides impliquées dans l'auto-réparation de la muqueuse (TGFβ, peptides à structure en trèfle), des peptides régulant les sécrétions épithéliales (guanyline) et enfin des agents régulateurs du système immunitaire local (cytokines). La composition cellulaire peut donc être utilisée en tant que système de production de ces molécules de manière à permettre l'extraction desdites molécules ainsi produites dans le milieu de culture et leur purification ultérieure. Il est donc ainsi possible, grâce à cette composition cellulaire, de mettre en oeuvre un procédé d'obtention de molécules biologiquement actives qui se caractérise par une culture de ladite composition cellulaire dans un milieu de culture approprié, tel que le milieu décrit ci-dessus, pendant un temps approprié, puis à extraire lesdites molécules du milieu de culture et à les purifier.

Cette même application peut également permettre l'obtention d'ARNm qui sont extraits desdites cellules. Un tel procédé d'obtention d'ARNm spécifiques de l'épithélium intestinal se caractérise par une étape de culture de la composition cellulaire dans un milieu approprié puis par une étape d'extraction desdits ARN des cellules par des techniques bien connues.

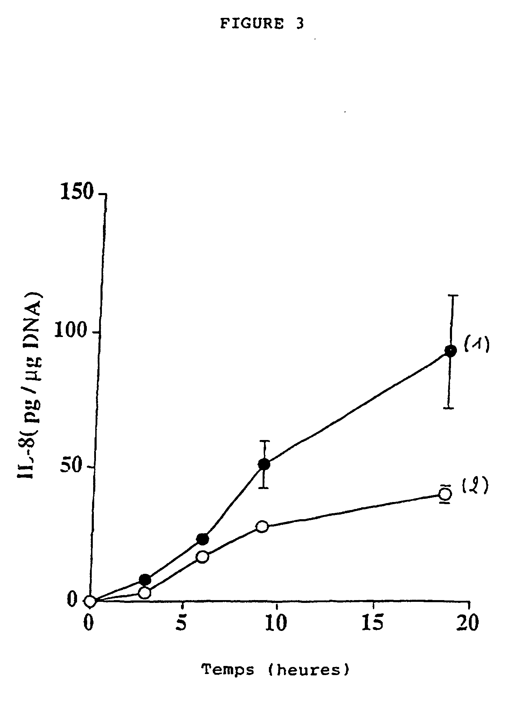

Ainsi, à titre d'exemple, la figure 3 illustre la production de cytokine (Interleukine-8) par des cellules épithéliales intestinales humaines normales obtenues conformément aux étapes 1 à 3 décrites ci-dessus, ces dites cellules épithéliales ayant été cultivées dans un milieu de culture tel que le milieu A décrit ci-dessus en présence (courbe 1) ou en l'absence (courbe 2) d'un agent pathogène.

L'agent pathogène introduit dans ce milieu de culture a permis d'induire une production d'Interleukine-8 significativement supérieure à celle de cellules non traitées.

Il apparaît donc désormais possible de produire de nouvelles molécules à partir de compositions cellulaires obtenues par la mise en oeuvre de l'invention en adaptant les milieux de culture à chaque molécule devant être produite.

Cette composition cellulaire, cultivée dans un ou plusieurs milieux de culture appropriés, peut également être utilisée pour la réalisation de modèle d'étude in vitro du comportement desdites cellules, notamment dans le domaine de la pharmacotoxicologie, de l'immunologie, en particulier pour l'aide à la fabrication de vaccins, et de la thérapie génique. Ceci est d'un grand intérêt, en particulier lorsque les cellules sont des cellules épithéliales intestinales.

Dans le cas où le procédé de culture est plus particulièrement appliqué à des lignées cellulaires cultivées en fermenteur sur des supports quelconques, tels que des mierobilles, les étapes essentielles du procédé de culture se décomposeront comme suit :
- ensemencement du milieu d'incubation, par exemple le milieu (MIA) (Kcl) décrit ci-dessus, dans le fermenteur avec des cellules de lignée à cultiver,
- agitation pendant 30 minutes environ à une température de l'ordre de 37°C,
- vidange du fermenteur et remplacement du milieu d'incubation par un milieu de culture quelconque, les cellules cultivées étant quant à elles adhérées aux microbilles disposées à l'intérieur du fermenteur.

## Revendications

1. Procédé de culture in vitro de cellules, telles que des cellules primaires normales ou tumorales et/ou des lignées cellulaires, d'origine humaine ou animale,
**caractérisé en ce qu'**il comprend, avant l'étape de mise en culture dans un milieu de culture apte à assurer la survie des cellules et à maintenir leurs fonctions spécialisées, au moins une étape d'incubation transitoire pendant une période de temps prédéterminée comprise dans la plage [5-60] minutes desdits cellules, de préférence isolées, dans un milieu d'incubation se présentant sous forme d'une solution dont les constituants sont choisis de manière telle que la concentration calcique finale de la solution est comprise dans la plage [0 - 1 mM], de préférence [0-100 µM] pour provoquer une adhérence rapide et en masse proche d'un rendement d'adhérence de 100 % desdites cellules isolées à un support quelconque en contact avec le milieu d'incubation.

2. Procédé de culture selon la revendication 1,
**caractérisé en ce que** les cellules, une fois adhérées à leur support, sont cultivées dans un milieu de culture dont la concentration calcique est progressivement augmentée jusqu'à une valeur prédéterminée apte à assurer la survie des cellules et à maintenir leurs fonctions spécialisées.

3. Procédé de culture selon l'une des revendications 1 et 2,
**caractérisé en ce que** le milieu d'incubation à pH et osmolarité contrôlées se présente sous forme d'une solution comprenant au moins un élément nutritif et un ou plusieurs sels choisis de manière telle qu'au moins l'un des cations est présent dans la solution à une concentration au moins égale à 50 % de la concentration cationique finale de ladite solution.

4. Milieu d'incubation de cellules, telles que des cellules primaires normales ou tumorales et/ou des lignées cellulaires d'origine humaine ou animale, pour la mise en oeuvre du procédé de culture selon l'une des revendications 1 à 3,
**caractérisé en ce que** le milieu, exempt de protéines d'origine animale, se présente sous forme d'une solution dont les constituants sont choisis de manière telle que la concentration calcique finale de la solution est comprise dans la plage [0 - 1 mM], de préférence [0 - 100 µM].

5. Milieu d'incubation selon la revendication 4,
**caractérisé en ce qu'**il se présente sous forme d'une solution comprenant au moins, outre un élément nutritif, tel que du glucose et/ou de la créatine, et éventuellement un antioxydant, tel que de la taurine, au moins un ou plusieurs sels, le ou lesdits sels étant choisis de manière telle qu'au moins l'un des cations est présent dans la solution à une concentration au moins égale à 50 % de la concentration cationique finale de ladite solution.

6. Milieu d'incubation selon l'une des revendications 4 et 5,
**caractérisé en ce qu'**il présente la composition suivante :
KCl : 30-90 mM
K-glutamate : 1-10 mM
KH₂PO₄ : 10-50 mM
MgSO₄ : 1-10 mM
CaCl₂ : 0-0,1 mM
EGTA : 0-1 mM
créatine : 1-10 mM
taurine : 1-30 mM
glucose : 5-30 mM
Hepes : 10 mM
pH ajusté à 7.2 avec KOH.

7. Milieu d'incubation selon l'une des revendications 4 et 5,
**caractérisé en ce qu'**il présente la composition suivante :
NaCl : 30-90 mM
Na-glutamate : 1-10 mM
NaH₂PO₄ : 10-50 mM
MgSO₄ : 1-10 mM
CaCl₂ : 0,01 mM
EGTA / 0-1 Mm
créatine : 1-10 mM
taurine : 1-30 mM
glucose : 5-30 mM
Hepes : 10mM
pH ajusté à 7,2 avec KOH.

8. Composition cellulaire obtenue par la mise en oeuvre du procédé selon l'une des revendications 1 à 3,
**caractérisée en ce qu'**elle est constituée d'une population pure de cellules épithéliales gastro-intestinales primaires normales adhérées en masse à un support avant d'être cultivées dans un milieu de culture, l'adhérence audit support ayant été obtenue par incubation transitoire pendant une période de temps comprise dans la plage [5 - 60 min] de primoculture de cellules épithéliales gastro-intestinales normales isolées de mammifères dans un milieu d'incubation se présentant sous forme d'une solution dont les constituants sont choisis de manière telle que la concentration calcique finale de la solution du milieu d'incubation est comprise dans la plage [0 - 1mM], de préférence [0 - 100 µM].

9. Procédé de production in vitro par l'épithélium gastro-intestinal de molécules biologiquement actives, telles que les facteurs de croissance, les facteurs de réparation et/ou d'ARNm codant pour lesdites molécules,
**caractérisé en ce qu'**il consiste à cultiver une composition cellulaire selon la revendication 8 dans un ou plusieurs milieux de culture appropriés, puis à extraire les ARN messagers des cellules de ladite composition cellulaire et/ou les molécules biologiquement actives du milieu de culture dans lequel est placée ladite composition cellulaire avant de purifier ces molécules.

10. Dispositif pour l'étude in vitro du comportement des cellules épithéliales intestinales notamment dans des domaines tels que la pharmacotoxicologie, l'immunologie, en particulier pour l'aide à la fabrication de vaccins, et la thérapie génique,
**caractérisé en ce qu'**il est constitué d'une composition cellulaire selon la revendication 8 cultivée dans un ou plusieurs milieux de culture appropriés.

## Patentansprüche

1. Verfahren zur In-Vitro-Kultivierung von Zellen, beispielsweise normalen Primärzellen oder Tumorzellen und/oder Zelllinien humanen oder tierischen Ursprunges, **dadurch gekennzeichnet, daß** es vor dem Schritt der Kultivierung in einem Kultivierungsmedium, das geeignet ist, das Überleben der Zellen zu sichern und ihre spezialisierten Funktionen aufrechtzuerhalten, wenigstens einen Schritt der vorübergehenden Inkubation der genannten Zellen während eines vorbestimmten Zeitraumes in dem Bereich [5 - 60] Minuten aufweist, wobei die Zellen vorzugsweise in einem Inkubationsmedium isoliert sind, das die Form einer Lösung aufweist, deren Bestandteile derart gewählt sind, daß die Kalzium-Endkonzentration der Lösung in dem Bereich [0 - 1 mM], vorzugsweise [0 - 100 µM] liegt, um eine schnelle Anhaftung und in der benachbarten Masse einen Anhaftungs-Wirkungsgrad von 100 % der genannten Zellen zu erzielen, die auf einem beliebigen Träger in Kontakt mit dem Inkubationsmedium gehalten sind.

2. Verfahren zur Kultivierung nach Anspruch 1,
**dadurch gekennzeichnet, daß** die Zellen, dann, wenn sie einmal an ihrem Träger anhaften, in einem Kultivierungsmedium kultiviert werden, dessen Kalziumkonzentration progressiv bis zu einem vorbestimmten Wert erhöht wird, der geeignet ist, das Überleben der Zellen sicherzustellen und ihre spezialisierten Funktionen aufrechtzuerhalten.

3. Verfahren zur Kultivierung nach einem der Ansprüche 1 und 2,
**dadurch gekennzeichnet, daß** das Inkubationsmedium mit einem eingestellten pH-Wert und einer eingestellten Osmolarität die Form einer Lösung aufweist, die wenigstens ein Nährelement und ein Salz oder mehrere Salze aufweist, die derart gewählt sind, daß wenigstens eines der Kationen in der Lösung mit einer Konzentration von wenigstens 50 % der Kalzium-Endkonzentration der genannten Lösung vorhanden ist.

4. Inkubationsmedium für Zellen, beispielsweise normalen Primärzellen oder Tumorzellen und/oder Zelllinien humanen oder tierischen Ursprunges, zur Durchführung des Kultivierungsverfahrens nach einem der Ansprüche 1 bis 3,
**dadurch gekennzeichnet, daß** das Medium, das von Proteinen tierischen Usprungs befreit ist, die Form einer Lösung aufweist, deren Bestandteile derart gewählt sind, daß die Kalzium-Endkonzentration der Lösung in dem Bereich [0 - 1 mM], vorzugsweise [0 - 100 µM] liegt.

5. Inkubationsmedium nach Anspruch 4,
**dadurch gekennzeichnet, daß** es in Form einer Lösung vorliegt, die neben einem Nährelement, beispielsweise Glukose und/oder Kreatin, und ggf. einem Antioxidationsmittel, beispielsweise Taurin, wenigstens ein Salz oder mehrere Salze aufweist, wobei das Salz oder die Salze derat gewählt ist bzw. sind, daß wenigstens eines der Kationen in der Lösung mit einer Konzentration vorhanden ist, die wenigstens 50 % der End-Kationenkonzentration der genannten Lösung entspricht.

6. Inkubationsmedium nach einem der Ansprüche 4 und 5,
**dadurch gekennzeichnet, daß** es die folgende Zusammensetzung aufweist:
KCL : 30-90 mM
K-Glutamat : 1-10 mM
KH₂PO₄ : 10-50 mM
MgSO₄ : 1-10 mM
CaCl₂ : 0-0,1 mM
EGTA : 0-1 mM
Kreatin : 1-10 mM
Taurin : 1-30 mM
Glukose : 5-30 mM
Hepes : 10 mM
pH-Wert eingestellt auf 7,2 mit KOH.

7. Inkubationsmedium nach einem der Ansprüche 4 und 5,
**dadurch gekennzeichnet, daß** es die folgende Zusammensetzung aufweist:
NaCl : 30-90 mM
Na-Glutamat : 1-10 mM
NaH₂PO₄ : 10-50 mM
MgSO₄ : 1-10 mM
CaCl₂ : 0,1 mM
EGTA / 0-1 mM
Kreatin : 1-10 mM
Taurin : 1-30 mM
Glukose : 5-30 mM
Hepes : 10mM
ph-Wert eingestellt auf 7,2 mit KOH.

8. Zellzusammensetzung, die durch Durchführung des Verfahrens nach einem der Ansprüche 1 bis 3 erhalten wird,
**dadurch gekennzeichnet, daß** sie durch eine Population aus normalen, primären, epithelialen, gastro-intestinalen Zellen besteht, die in einer Menge an einem Träger anhaften, bevor sie in einem Kultivierungsmedium kultiviert werden, wobei die Anhaftung an den genannten Träger durch vorübergehende Inkubation während eines Zeitraumes im Bereich [5 - 60 min] der Primärkultur der epithelialen, gastro-intestinalen normalen Zellen erhalten wird, die aus Säugern isoliert werden in einem Inkubationsmedium, das die Form einer Lösung aufweist, deren Bestandteile derart gewählt sind, daß die Kalzium-Endkonzentration der Lösung des Inkubationsmediums in dem Bereich [0 - 1 mM], vorzugsweise [0-100 µM] liegt.

9. Verfahren zur In-Vitro-Herstellung von biologisch aktiven Molekülen durch das gastro-intestinale Epithel, beispielsweise von Wachstumsfaktoren, Reparaturfaktoren und/oder RNA-Boten, die für die genannten Molikülen codierend sind,
**dadurch gekennzeichnet, daß** es in einer Kultivierung einer Zellzusammensetzung nach Anspruch 8 in einem geeigneten Kultivierungsmedium oder mehreren geeigneten Kultivierungsmedien und daran anschließend in einem Kultivieren der RNA-Boten der Zellen der genannten Zellzusammensetzung und/oder der biologisch aktiven Moleküle aus dem Kultivierungsmedium besteht, in dem die genannte Zusammensetzung vor der Reinigung dieser Moliküle bereitgestellt wurde.

10. Vorrichtung für das In-Vitro-Studium des Verhaltens von intestinalen Epithel-Zellen, insbesondere im Bereich der Pharmatoxikologie und Immunologie, insbesondere zur Unterstützung der Herstellung von Vakzinen, und der Gentherapie,
**dadurch gekennzeichnet, daß** sie durch eine Zellzusammensetzung nach Anspruch 8 gebildet ist, die in einem geeigneten Kultivierungsmedium oder mehreren geeigneten Kultivierungsmedien kultiviert wird.

## Claims

1. Culture method, in vitro, for growing cells such as normal or tumoral primary cells and/or cellular lineages, of human or animal origin, **characterised in that** it comprises, prior to the stage of starting the culture in a culture medium capable of ensuring the survival of the cells and of maintaining their specialised functions, at least one temporary incubation stage, for a predetermined period of time within the range [5 - 60] minutes, of said cells, preferably isolated, in an incubation medium in the form of a solution, the constituents of which are selected in such a manner that the final calcic concentration of the solution is within the range [0 - 1 mM], preferably [0 - 100 µM], to cause a rapid, en masse adherence, close to an adherence yield of 100 %, of said isolated cells to any support in contact with the incubation medium.

2. Culture method according to claim 1, **characterised in that** the cells, once they have adhered to their support, are grown in a culture medium, the calcic concentration of which is progressively increased up to a predetermined value which is capable of ensuring the survival of the cells and of maintaining their specialised functions.

3. Culture method according to one of claims 1 and 2, **characterised in that** the incubation medium, with controlled pH and osmolarity, is in the form of a solution comprising at least one nutritive element and one or more salts selected in such a manner that at least one of the cations is present in the solution at a concentration at least equal to 50 % of the final cationic concentration of said solution.

4. Incubation medium for cells, such as normal or tumoral primary cells and/or cellular lineages, of human or animal origin, for accomplishing the culture method according to one of claims 1 to 3, **characterised in that** the medium, exempt from proteins of animal origin, is in the form of a solution, the constituents of which are selected in such a manner that the final calcic concentration of the solution is within the range [0 - 1 mM], preferably [0 - 100 µM].

5. Incubation medium according to claim 4, **characterised in that** it is in the form of a solution comprising, in addition to a nutritive element such as glucose and/or creatine, and possibly an anti-oxidising agent such as taurine, at least one or more salts, the said salt or salts being selected in such a manner that at least one of the cations is present in the solution at a concentration at least equal to 50 % of the final cationic concentration of said solution.

6. Incubation medium according to one of claims 4 and 5, **characterised in that** it has the following composition:
KCl: 30-90 mM
K-glutamate: 1-10 mM
KH₂PO₄: 10-50 mM
MgSO₄: 1-10 mM
CaCl₂: 0-0.1 mM
EGTA: 0-1 mM
creatine: 1-10 mM
taurine: 1-30 mM
glucose: 5-30 mM
Hepes: 10 mM
pH adjusted to 7.2 with KOH.

7. Incubation medium according to one of claims 4 and 5, **characterised in that** it has the following composition:
NaCl: 30-90 mM
Na-glutamate: 1-10 mM
NaH₂PO₄: 10-50 mM
MgSO₄: 1-10 mM
CaCl₂: 0.01 mM
EGTA: 0-1 mM
creatine: 1-10 mM
taurine: 1-30 mM
glucose: 5-30 mM
Hepes: 10 mM
pH adjusted to 7.2 with KOH.

8. Cellular composition obtained by accomplishing the method according to one of claims 1 to 3, **characterised in that** it is made up of a pure population of normal primary gastro-intestinal epithelial cells adhered en masse to a support before being grown in a culture medium, the adherence to said support having been obtained by temporary incubation for a period of time within the primoculture range [5 - 60 min] of isolated normal gastro-intestinal epithelial cells of mammals in an incubation medium in the form of a solution, the constituents of which are selected in such a manner that the final calcic concentration of the solution of the incubation medium is within the range [0 - 1 mM], preferably [0 - 100 µM].

9. In vitro production method through the gastro-intestinal epithelium of biologically active molecules, such as growth factors, repair and/or mRNA coding for said molecules, **characterised in that** it comprises growing a cellular composition according to claim 8 in one or more appropriate culture media, then extracting the mRNA carriers of the cells from said cellular composition and/or the biologically active molecules from the culture medium in which said cellular composition is placed before purifying these molecules.

10. Apparatus for the in vitro study of the behaviour of intestinal epithelial cells, more especially in fields such as pharmaco-toxicology, immunology, in particular for assisting in the manufacture of vaccines, and gene therapy, **characterised in that** it is made up of a cellular composition according to claim 8, grown in one or more appropriate culture media.
